# EUROPEAN PATENT SPECIFICATION

(11) **EP 2 830 434 B1**
(45) Date of publication and mention of the grant of the patent: **16.03.2016**
(21) Application number: 13714000.0
(22) Date of filing: 21.03.2013
(51) Int. Cl.: A23K 50/00, A23K 20/00

(54) **USE OF PARA NITRO AMINO DERIVATIVES IN FEED FOR REDUCING ME-THANE EMISSION IN RUMINANTS**
VERWENDUNG VON PARANITRO-AMINO-DERIVATEN BEI DER ZUFUHR ZUR REDUKTION DER METHANEMISSION BEI WIEDERKÄUERN
UTILISATION DE DÉRIVÉS PARA NITRO AMINÉS DANS DES ALIMENTS POUR RÉDUIRE L'ÉMISSION DE MÉTHANE CHEZ LES RUMINANTS

(30) Priority: 26.03.2012 EP 12161270
(43) Date of publication of application: 04.02.2015
(73) Proprietor: DSM IP Assets B.V., 6411 TE Heerlen (NL)
(72) Inventor: DUVAL, Stephane, F-68305 Saint-Louis Cedex (FR); KINDERMANN, Maik, CH-4002 Basel (CH)
(74) Representative: Barthelemy de Saizieu, Antoine
(86) International application number: PCT/IB2013/052230
(87) International publication number: WO 2013/144786

(56) References cited:
- WO-A1-2011/045418
- WO-A1-2011/070133
- US-A1- 2003 219 467

## Description

The present invention relates to the use of at least one compound belonging to the class of para nitro amino derivatives for reducing the production of methane emanating from the digestive activities of ruminants.

The present invention also relates to animal feed or animal feed compositions and feed additives comprising the above mentioned compounds. The term feed or feed composition means any compound, preparation, mixture, or composition suitable for, or intended for intake by an animal.

In the present context, a ruminant is a mammal of the order Artiodactyla that digests plant-based food by initially softening it within the animal's first stomach, known as the rumen, then regurgitating the semi-digested mass, now known as cud, and chewing it again. The process of again chewing the cud to further break down plant matter and stimulate digestion is called "ruminating".

Rumen fermentation brings some disadvantages. Methane is produced as a natural consequence of the anaerobic fermentation, which represents an energy loss to the host animal. Carbohydrate makes up 70 - 80% of the dry matter in a typical dairy cattle ration and in spite of this the absorption of carbohydrates from the gastrointestinal tract is normally very limited. The reason for this is the extensive fermentation of carbohydrates in the rumen resulting in production of acetate, propionate and butyrate as the main products. These products are part of the so called volatile fatty acids, (VFAs).

Besides the energy loss, methane is also a greenhouse gas, which is many times more potent than CO₂. Its concentration in the atmosphere has doubled over the last century and continues to increase alarmingly. Ruminants are the major contributors to the biogenic methane formation, and it has been estimated that the prevention of methane formation from ruminants would almost stabilize atmospheric methane concentrations.

Furthermore, the assessment of the Kyoto protocol followed by the Copenhagen climate summit in 2009 places increased priority in decreasing methane emissions as part of a multi-gas strategy. The most effective additives currently used for reducing the formation of methane contain antibiotics which diminish the proliferation of microorganisms providing hydrogen (H₂) to the methanogens (Sauer et al. 1998. American Society of Animal Science; 76: 906-914). However, the effect of antibiotics on the formation of methane has some disadvantages because of rapid adaptation of the microflora and/or resistance development leading to a complete loss of the intended effect within a short period of time (2 to 3 weeks), and because the use of antibiotics is banned in Europe for non therapeutic use.

Non antibiotic products (bile acid derivatives) leading to reduction of methane emission, when tested using an *in vitro* rumen simulation model, have recently been published (WO 2010072584). However, the amount required to produce a moderate reduction of methane emission are not compatible with the ruminant feed industry cost constraints.

Under these circumstances there is still a need to develop new substances which reduce the formation of methane and which are in line with reliable and generally accepted practice and not of a medicinal nature. In addition to reducing methane emission, such substances may also contribute to improve ruminant performance by improving the feed conversion ratio, reducing feed intake, improving weight gain, and/or improving carcass, or milk yield.

The present inventors now surprisingly found that the compounds specified herein after, have a great potential for use in animal feed in order to essentially reduce the formation of methane without affecting microbial fermentation in a way that would be detrimental to the host animal. Moreover, the compounds of the present invention may have a great benefit regarding overall animal performance as measured by feed conversion ratio, feed intake, weight gain, carcass yield, or milk yield. Said compounds are also more stable than those described in the prior art, safer for the animal and human, lead to persistent methane reduction effect, they do not affect palatability, they can be produced at industrial scale at a cost compatible with the animal nutrition industry, and above all, they do not provoke accumulation of any metabolite in the milk or meat of the supplemented animal, and they are active at very low concentration in the rumen.

In particular, the present inventors have observed that the feeding to ruminants of at least one para nitro amino derivative compound is very effective in reducing the production of methane emanating from the digestive activities of ruminants without negatively affecting total VFA production.

Moreover, the present inventors have shown that when the amine function is replaced, or when the para position of the nitro group is modified to meta-, the technical effect on methane production is lost.

Therefore, the present invention provides the use of at least one compound as defined by formula (I), or a salt thereof as an active compound in animal feeding for reducing the formation of methane emanating from the digestive activities of ruminants. 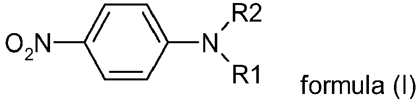 wherein R¹ and R² independently of each other represent H, -CH₃, or -CH₂R³, and wherein R³ is a saturated or unsaturated, linear, branched or cyclic C₁-C₈ - hydrocarbon group, optionally substituted with 1 to 3 groups selected from -OH,-NH₂, -COOH, and wherein one or two of the carbon atoms in the C₁-C₈ hydrocarbon is optionally substituted by a nitrogen or an oxygen atom.

The invention further provides a method for reducing the production of methane emanating from the digestive activities of ruminants comprising orally administering a sufficient amount of at least one para nitro amino derivative, or a salt thereof as defined by formula (I) to the animal. It is to be understood by oral administration a simple feeding, or manual administration of a bolus.

In all embodiments of the present invention, the compound of formula (I) or salts thereof are defined by the following compound of formula (I) 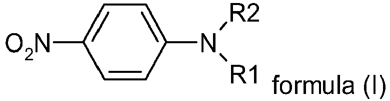 wherein R¹ and R² independently of each other represent H, -CH₃, or -CH₂R³, and wherein R³ is a saturated or unsaturated, linear, branched or cyclic C₁-C₈ - hydrocarbon group, optionally substituted with 1 to 3 groups selected from -OH,-NH₂, -COOH, and wherein one or two of the carbon atoms in the C₁-C₈ hydrocarbon is optionally substituted by a nitrogen or an oxygen atom.

In another embodiment, preferred compounds of formula (I) according to the present invention are compounds, wherein R¹ is H or -CH₃. More preferred compounds of formula (I) are compounds wherein R¹ is H or -CH₃, R² is H, -CH₃, or -CH₂R³, and R³ is a saturated or unsaturated, linear, or branched C₁-C₄ -hydrocarbon group. Even more preferred compounds of formula (I) are compounds wherein R¹ is H or-CH₃, R² is H, -CH₃, or -CH₂R³, and R³ is a saturated, linear, or branched C₁-C₃ - hydrocarbon group.

Most preferred compounds according to the present invention are selected from the group of compounds comprising (4-nitro-phenyl)-amine, methyl-(4-nitro-phenyl)-amine, ethyl-(4-nitro-phenyl)-amine, dimethyl-(4-nitro-phenyl)-amine, (4-Nitro-phenyl)-propyl-amine, isopropyl-(4-nitro-phenyl)-amine, 2-(4-Nitro-phenylamino)-ethanol, 3-(4-Nitro-phenylamino)-propan-1-ol, 4-Nitro-N-(Pyridin-2-ylmethyl)aniline, 4-((4-Nitro-phenylamino)-methyl)-benzoic acid, and 3-((4-Nitro-phenylamino)-methyl)-benzoic acid as listed and depicted in table 1.

**Table 1: Preferred compounds of formula (I) according to the present invention**

| **Compound Identifier** | **Compound structure** | **Compound name** |
|---|---|---|
| 1 | 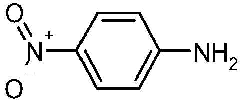 | (4-nitro-phenyl)-amine |
| 2 | 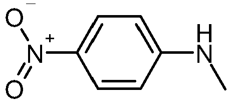 | Methyl-(4-nitro-phenyl)-amine |
| 3 | 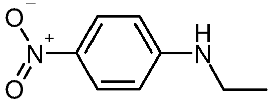 | Ethyl-(4-nitro-phenyl)-amine |
| 4 | 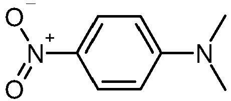 | Dimethyl-(4-nitro-phenyl)-amine |
| 5 | 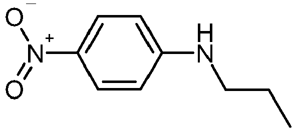 | (4-Nitro-phenyl)-propylamine |
| 6 | 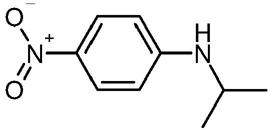 | Isopropyl-(4-nitro-phenyl)-amine |
| 7 | 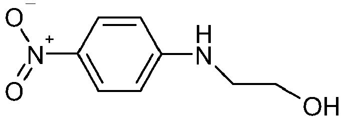 | 2-(4-Nitro-phenylamino)-ethanol |
| 8 | 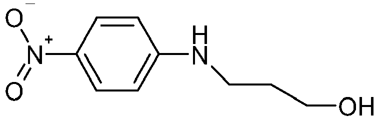 | 3-(4-Nitro-phenylamino)-propan-1-ol |
| 9 | 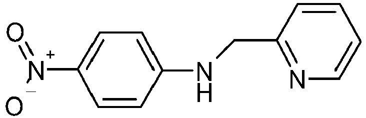 | 4-Nitro-N-(Pyridin-2-ylmethyl)aniline |
| 10 | 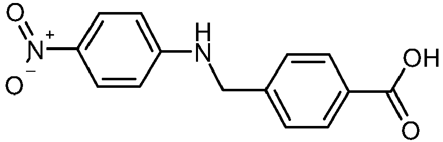 | 4-((4-Nitro-phenylamino)-methyl)-benzoic acid |
| 11 | 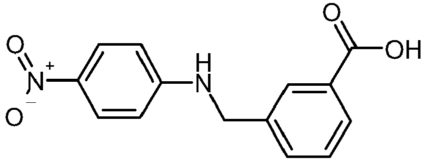 | 3-((4-Nitro-phenylamino)-methyl)-benzoic acid |

For all embodiments of the present invention, it is to be understood that compounds of formula (I) can be in any isomeric form.

The compounds of the present invention also comprise salts of the compoundd of formula (I). Preferred cations for salt preparation may be selected from the group consisting of sodium (Na+), potassium (K+), lithium (Li+), magnesium (Mg2+), calcium (Ca2+), barium (Ba2+), strontium (Sr2+), and ammonium (NH4+). Salts may also be prepared from an alkali metal or an alkaline earth metal.

The compounds of the present invention can be manufactured in principle according to synthetic methods known *per se* in the art, and/or based on methods as described in the examples.

In all these cases appropriate methods to purify the product (compounds of formula (I)) can be chosen by those skilled in the art, i.e. by column chromatography, or the compound of formula (I), can be isolated and purified by methods known per se.

Methane emission by ruminants can easily be measured in individual animals in metabolic chambers by methods known in the art (Grainger et al., 2007 J. Dairy Science; 90: 2755-2766). Moreover, it can also be assessed at barn level by an emerging technology using laser beam (McGinn et al., 2009, Journal of Environmental Quality; 38: 1796-1802). Alternatively, methane produced by a dairy ruminant can also be assessed by measurement of VFA profiles in milk according to WO 2009/156453.

Ruminant performance can be assessed by methods well known in the art, and is usually characterized by feed conversion ratio, feed intake, weight gain, carcass yield, or milk yield.

The present invention also relates to the use of a compound of formula (I), or a salt thereof according to the present invention in combination with at least one additional active substance which shows similar effects with regard to methane formation in the rumen and which is selected from the group consisting of diallyl disulfide, garlic oil, allyl isothiocyanate, deoxycholic acid, chenodeoxycholic acid and derivatives thereof.

Further components that could be given together with the compound according to the present invention are for example yeasts, essential oils, and ionophores like monensin, rumensin.

It is at present contemplated that diallyl disulfide, garlic oil, allyl isothiocyanate deoxycholic acid, chenodeoxycholic acid and derivatives thereof are independently administered in dosage ranges of for example 0.01-500 mg active substance per kg feed (ppm). These compounds are either commercially available or can easily be prepared by a skilled person using processes and methods well-known in the prior art.

Ruminating mammals according to the present invention include cattle, goats, sheep, giraffes, American Bison, European bison, yaks, water buffalo, deer, camels, alpacas, Ilamas, wildebeest, antelope, pronghorn, and nilgai.

For all embodiments of the present invention, cattle, goats, sheep, American Bison, European bison, yaks, and water buffalo are the preferred species. More preferred species are domestic cattle, sheep and goat, most preferred species are domestic cattle. The term includes all races of domestic cattle, and all production kinds of cattle, in particular dairy cows and beef cattle.

The present invention also relates to the use of at least one compound of formula (I), or a salt thereof according to the present invention, wherein the methane production in ruminants calculated in liters per kilogram of dry matter intake is reduced by at least 10 % when measured in metabolic chambers. Preferably, methane reduction is at least 15 %, more preferably, at least 20 %, even more preferably, at least 25 %, most preferably, at least 30 %. Alternative methane emission measurements may also be used like using a laser beam or for dairy ruminants, correlating methane production to the VFA profile in milk.

The present invention also relates to the use of at least one compound of formula (I), or a salt thereof according to the present invention, wherein the ruminant feed conversion ratio is reduced by at least 1 % when measured in conventional performance trial. Preferably, the feed conversion ratio is reduced by at least 2 %, more preferably, by at least 2.5 %, even more preferably, by at least 3 %, most preferably, by at least 3.5 %.

The present invention also relates to the use of at least one compound of formula (I), or a salt thereof according to the present invention, wherein the amount of the compound of formula (I) administered to the ruminant animal is from 1 mg to 10 g per Kg of feed, preferably from 10 mg to 1 g per Kg of feed, more preferably, from 50 mg to 500 mg per Kg of feed. For the use in animal feed, however, compounds of formula (I), or their salts thereof need not be that pure; it may e.g. include other compounds and derivatives.

As indicated above, the compounds of the present invention are useful as compounds for feed additives and animal feed compositions for ruminants, and accordingly are useful as the active ingredients in such feed to reduce methane formation in the digestive tract of the animal.

For the realisation of their use as such ingredients for the feed of ruminants the compounds may be incorporated in the feed by methods known per se in the art of feed formulation and processing.

Further aspects of the present invention are therefore formulations, i.e. feed additives and animal feed compositions containing compounds as herein above defined. The present invention therefore also relates to a feed composition or a feed additive comprising at least one compound of formula (I) or a salt thereof. In a preferred embodiment, the composition is a mineral premix, a vitamin premix including vitamins and minerals or a bolus.

The normal daily dosage of a compound according to the invention provided to an animal by feed intake depends upon the kind of animal and its condition. Normally this dosage should be in the range of from about 1 mg to about 10 g, preferably from about 10 mg to about 1 g, more preferably, 50 mg to 500 mg compound per kg of feed.

The compound of formula (I), or a salt thereof may be used in combination with conventional ingredients present in an animal feed composition (diet) such as calcium carbonates, electrolytes such as ammonium chloride, proteins such as soya bean meal, wheat, starch, sunflower meal, corn, meat and bone meal, amino acids, animal fat, vitamins and trace minerals.

Particular examples of compositions of the invention are the following:
- An animal feed additive comprising (a) at least one compound selected from table 1 and (b) at least one fat-soluble vitamin, (c) at least one water-soluble vitamin, (d) at least one trace mineral, and/or (e) at least one macro mineral;
- An animal feed composition comprising at least one compound selected from table 1 and a crude protein content of 50 to 800 g/kg feed.

The so-called premixes are examples of animal feed additives of the invention. A premix designates a preferably uniform mixture of one or more micro-ingredients with diluents and/or carrier. Premixes are used to facilitate uniform dispersion of micro-ingredients in a larger mix.

Apart from the active ingredients of the invention, the premix of the invention contains at least one fat-soluble vitamin, and/or at least one water soluble vitamin, and/or at least one trace mineral, and/or at least one macro mineral. In other words, the premix of the invention comprises the at least one compound according to the invention together with at least one additional component selected from the group consisting of fat-soluble vitamins, water-soluble vitamins, trace minerals, and macro minerals.

Macro minerals may be separately added to the feed. Therefore, in a particular embodiment, the premix comprises the active ingredients of the invention together with at least one additional component selected from the group consisting of fat-soluble vitamins, water-soluble vitamins, and trace-minerals.

The following are non-exclusive lists of examples of these components:
- Examples of fat-soluble vitamins are vitamin A, vitamin D3, vitamin E, and vitamin K, e.g. vitamin K3.
- Examples of water-soluble vitamins are vitamin B12, biotin and choline, vitamin B1, vitamin B2, vitamin B6, niacin, folic acid and panthothenate, e.g. Ca-D-panthothenate.
- Examples of trace minerals are manganese, zinc, iron, copper, iodine, selenium, and cobalt.
- Examples of macro minerals are calcium, phosphorus and sodium.

As regards feed compositions for ruminants such as cows, as well as ingredients thereof, the ruminant diet is usually composed of an easily degradable fraction (named concentrate) and a fiber-rich less readily degradable fraction (named hay, forage, or roughage).

Hay is made of dried grass, legume or whole cereals. Grasses include among others timothy, ryegrasses, fescues. Legumes include among others clover, lucerne or alfalfa, peas, beans and vetches. Whole cereals include among others barley, maize (corn), oat, sorghum. Other forage crops include sugarcane, kales, rapes, and cabbages. Also root crops such as turnips, swedes, mangles, fodder beet, and sugar beet (including sugar beet pulp and beet molasses) are used to feed ruminants. Still further crops are tubers such as potatoes, cassava and sweet potato. Silage is an ensiled version of the fiber-rich fraction (e.g. from grasses, legumes or whole cereals) whereby material with a high water content is treated with a controlled anaerobic fermentation process (naturally-fermented or additive treated).

Concentrate is largely made up of cereals (such as barley including brewers grain and distillers grain, maize, wheat, sorghum), but also often contain protein-rich feed ingredients such as soybean, rapeseed, palm kernel, cotton seed and sunflower. Cows may also be fed total mixed rations (TMR), where all the dietary components, e.g. forage, silage and concentrate, are mixed before serving.

As mentioned above a premix is an example of a feed additive which may comprise the active compounds according to the invention. It is understood that the compounds may be administered to the animal in different other forms. For example the compounds can also be included in a bolus that would be placed in the rumen and that would release a defined amount of the active compounds continuously in well defined dosages over a specific period of time.

The present invention further relates to a method for reducing the production of methane emanating from the digestive activities of ruminants comprising orally administering a sufficient amount of at least one compound of formula (I), or a salt thereof with the preferred embodiments described above.

Moreover, the invention further relates to a method as described above, wherein the compound of formula (I) is administered to the animal in combination with at least one additional active substance selected from the group consisting of diallyl disulfide, garlic oil, allyl isothiocyanate, deoxycholic acid, chenodeoxycholic acid and derivatives thereof.

The invention also relates to a method as described above, wherein the ruminant animal is selected from the group consisting of: cattle, goats, sheep, giraffes, American Bison, European bison, yaks, water buffalo, deer, camels, alpacas, Ilamas, wildebeest, antelope, pronghorn, and nilgai, and more preferably from the group consisting of: cattle, goats and sheep.

The invention also relates to a method as described above, wherein the amount of the at least one active compound as defined in formula (I) administered to the ruminant animal is from about 1 mg to about 10 g per kg feed, preferably from about 10 mg to about 1 g, more preferably from 50 mg to 500 mg compound per kg of feed.

The invention also relates to a method as described above, wherein the methane production in ruminants calculated in liters per kilogram of dry matter intake is reduced by at least 10 % when measured in metabolic chambers. Preferably, methane reduction is at least 15 %, more preferably, at least 20 %, even more preferably, at least 25 %, most preferably, at least 30 %. Alternative methane emission measurements may also be used like using a laser beam or for dairy ruminants, correlating methane production to the VFA profile in milk.

The invention also relates to a method as described above, wherein the ruminant feed conversion ratio is reduced by at least 1 % when measured in conventional performance trial. Preferably, the feed conversion ratio is reduced by at least 2 %, more preferably, by at least 2.5 %, even more preferably, by at least 3 %, most preferably, by at least 3.5 %.

The present invention is further described by the following examples which should not be construed as limiting the scope of the invention.

### Examples

### Example 1: In vitro test for methane production

A modified version of the "Hohenheim Forage value Test (HFT)" was used for testing the effect of specific compounds on the rumen functions mimicked by this in-vitro system.

### Principle:

Feed is given into a syringe with a composition of rumen liquor and an appropriate mixture of buffers. The solution is incubated at 39 °C. After 8 hours the quantity (and composition) of methane produced is measured and put into a formula for conversion.

### Reagents:

### Mass element solution:

- 6.2 g potassium dihydrogen phosphate (KH₂PO₄)
- 0.6 g magnesium sulfate heptahydrate (MgSO₄ * 7H₂O)
- 9 ml concentrated phosphoric acid (1 mol/l)
- dissolved in distilled water to 1 l (pH about 1.6)

### Buffer solution:

- 35.0 g sodium hydrogen carbonate (NaHCO₃)
- 4.0 g ammonium hydrogen carbonate ((NH₄)HCO₃)
- dissolved in distilled water to 1 l

### Trace element solution:

- 13.2 g calcium chloride dihydrate (CaCl₂ * 2H₂O)
- 10.0 g manganese(II) chloride tetrahydrate (MnCl₂ * 4H₂O)
- 1.0 g cobalt(II) chloride hexahydrate (CoCl₂ * 6H₂O)
- 8.0 g iron(III) chloride (FeCl₃ * 6H₂O)
- dissolved in distilled water to 100 ml

### Sodium salt solution:

- 100 mg sodium salt
- dissolved in distilled water to 100 ml

### Reduction solution:

- first 3 ml sodium hydroxide (c = 1 mol/l), then 427.5 mg sodium sulfide hydrate (Na₂S * H₂O) are added to 71.25 ml H₂O
- solution must be prepared shortly before it is added to the medium solution

### Procedure:

### Sample weighing:

The feed stuff is sieved to 1mm - usually TMR (44 % concentrate, 6 % hay, 37 % maize silage and 13 % grass silage) - and weighed exactly into 64 syringes. 4 of these syringes are the substrate controls, which display the gas production without the effect of the tested compounds. 4 other syringes are positive control, in which bromoethane sulfonate has been added to 0.1 mM. When needed, 4 syringes contain a carrier control (if the test compounds need a carrier). The remaining syringes contain the test substances, by groups of 4 syringes.

### Preparation of the medium solution:

The components are mixed in a Woulff bottle in following order:
- 711 ml water
- 0.18 ml trace element solution
- 355.5 ml buffer solution
- 355.5 ml mass element solution

The completed solution is warmed up to 39 °C followed by the addition of 1.83 ml sodium salt solution and the addition of reduction solution at 36 °C. The rumen liquor is added, when the indicator turns colourless.

### Extraction of the rumen liquor:

750 ml of rumen liquor are added to approximately 1,400 ml of medium solution under continued agitation and CO₂-gassing.

### Filling the syringes, incubation and determining gas volumes and VFA values:

The diluted rumen fluid (24 ml) is added to the glass syringe. The syringes are then incubated for 8 hours at 39 °C under gentle agitation. After 8 hours, the volume of gas produced is measured, and the percentage of methane in the gas phase is determined by gas chromatography.

### Results

The food fermented was artificial TMR (44 % concentrate, 6 % hay, 37 % maize silage and 13 % grass silage). The compounds produced as described in examples 3 to 11 were added to the fermentation syringes to a concentration of 1 to 0.1 % of dry matter (DM). The results of the in vitro effect are presented in the following table.

**Table 2: Methane reduction effect resulting from the average of two experiments with some compounds according to the present invention (an integer in the column effect on methanogenesis change (%) means a reduction in methane produced when compared to control; no value means that the concentration was not tested)**

| **Structure** | **effect on methanogenesis (%)** | | | |
|---|---|---|---|---|
| | 1 % DM | 0.5 % DM | 0.25 % DM | 0.1 % DM |
| 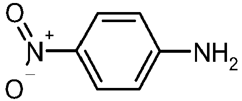 | 100 | 88 | 43 | 15 |
| 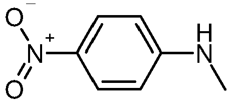 | 96 | | | 3 |
| 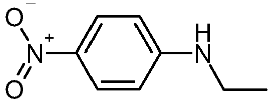 | 97 | | | 0 |
| 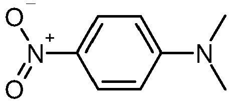 | 94 | | | 0 |
| 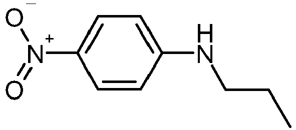 | 100 | 60 | 20 | 5 |
| 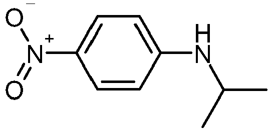 | 99 | | | 2 |
| 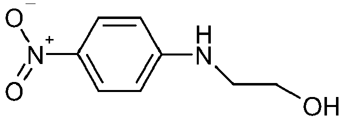 | 100 | | | 6 |
| 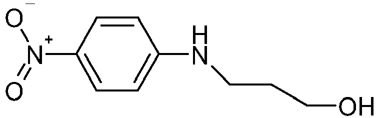 | 100 | | | 1 |
| 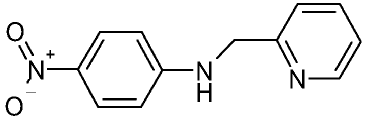 | 95 | | | 3 |
| 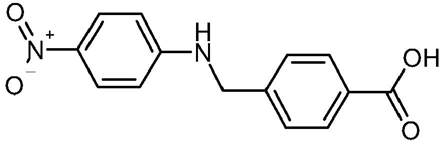 | 97 | 21 | 3 | |
| 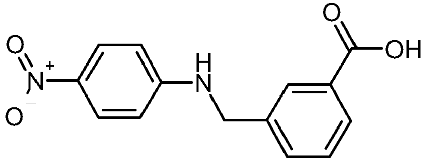 | 88 | | | 1 |

### Example 2: Comparative example: in vitro test for methane production.

The same *in vitro* assay as described in Example 1 has been performed with a series of molecules, wherein either the amine group has been replaced or, the amine group is not in para position. This data demonstrates that a significant methane reduction activity is only observed when an amino group is present and when it is in the para position (compare table 2 with table 3).

**Table 3: Methane reduction effect resulting from the average of two experiments with the below mentioned compounds in which the amino group has been exchanged, or wherein the position of the amino group has been exchanged. (An integer in the column effect on methanogenesis change (%) means a reduction in methane produced when compared to control; no value means that the concentration was not tested).**

| **ID** | **Structure** | **effect on methanogenesis (%)** | |
|---|---|---|---|
| | | 1 % DM | 0.1 % DM |
| A | 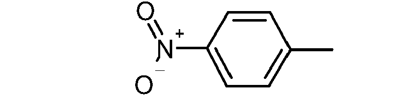 | 8 | |
| B | 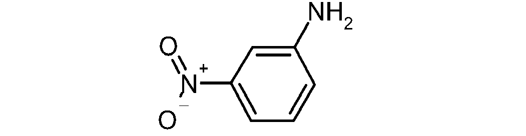 | 7 | |
| C | 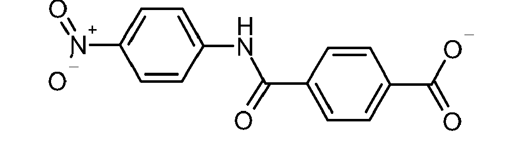 | 3 | |
| D | 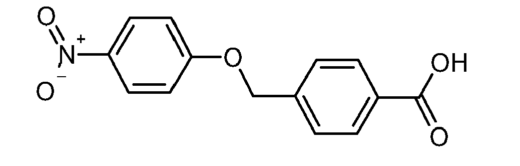 | 6 | |
| E | 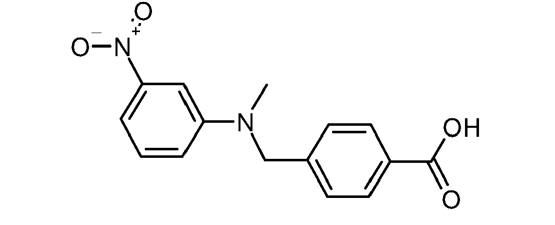 | 19 | |
| F | 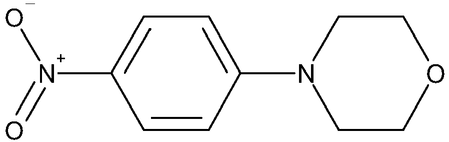 | 8 | |

For this comparative example, the compounds of table 3 were purchased from chemical suppliers or produced according to the following processes: **Compound B:** 3-Nitro-phenylamine

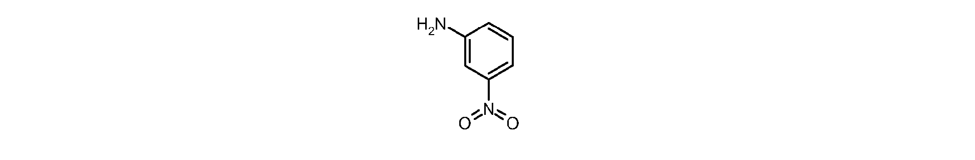

3-Nitro-phenylamine was purchased from Lancaster. CAS [99-09-2]. Catalogue # 4063. Batch # 000084111H. NMR (300 MHz, DMSO-d6): 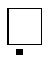 5.79 (broad s, 2H), 6.89-6.96 (m, 1H), 7.19-7.31 (m, 2H), 7.35 (t, J=2.0 Hz, 1H). MS: 139.0 [C6H6N2O2+H]⁺.

### Compound D: Synthesis of 4-(4-Nitro-phenoxymethyl)-benzoic acid

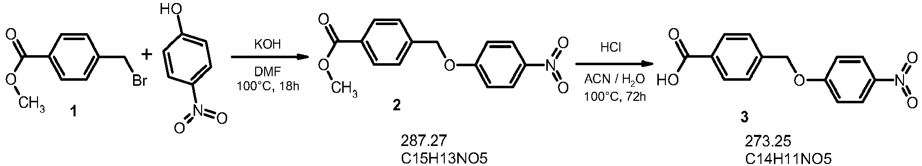

4-(4-Nitro-phenoxymethyl)-benzoic acid methyl ester 2:

To a suspension of potassium hydroxyde (1.70 g, 2.0 eq) in anhydrous dimethylformamide (100 mL) were added 4-nitrophenol (2.53 g, 1.2 eq) and methyl 4-(bromomethyl)benzoate **1** (3.48 g, 1.0 eq). The reaction mixture was stirred at 100°C for 18 hours. After water addition (200 mL), the mixture was extracted with ethyl acetate (3x100 mL), dried over magnesium sulphate and solvents were evaporated under vaccum. The residual suspension (in dimethylformamide) was filtered off and residual solvents evaporated in vacuo to afford compound **2** as a yellowish powder; yield 2.98 g (10.37 mmol, 68 %).
4-(4-Nitro-phenoxymethyl)-benzoic acid **3:**

To a solution of compound **2** (2.98 g, 1.0 eq) in acetonitrile (200 mL), was added a (3N) solution of hydrochloric acid (200 mL). The reaction mixture was stirred at 100°C for 72 hours. The resulting precipitate was filtered off to afford compound **3** as a white powder; yield 1.33 g (4.87 mmol, 47 %).
1H NMR (300 MHz, DMSO-d6): □ 5.35 (s, 2H), 7.22 (d, J=9.3 Hz, 2H), 7.56 (d, J=8.4 Hz, 2H), 7.96 (d, J=8.4 Hz, 2H), 8.21 (d, J=9.3 Hz, 2H), 12.97 (broad s, 1 H). MS: 274.1 [C14H11NO5+H]⁺.

### Compound F: 4-(4-Nitrophenyl)morpholine

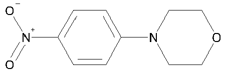

4-(4-Nitrophenyl)morpholine was purchased from ABCR. CAS [10389-51-2]. Catalogue # AV24933/AB146794. Batch # 1196165.

### Example 3: Synthesis of Methyl-(4-nitro-phenyl)-amine

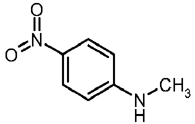

Methyl-(4-nitro-phenyl)-amine was purchased from Alfa Aesar. CAS [100-15-2]. Catalogue # A15548. Batch # A6950A. 1H NMR (300 MHz, DMSO-d6): 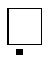 2.77 (d, J=5.0 Hz, 3H), 6.58 (d, J=9.3 Hz, 2H), 7.27 (broad s, 1 H), 7.98 (d, J=9.3 Hz, 2H). MS: 153.0 [C7H8N202+H]⁺.

### Example 4: Synthesis of Ethyl-(4-nitro-phenyl)-amine

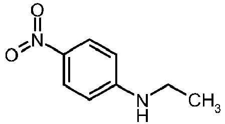

Ethyl-(4-nitro-phenyl)-amine was purchased from Sigma-Aldrich. CAS [3665-80-3]. Catalogue # 328499. Batch # 1061MC. 1H NMR (300 MHz, CHC13-d): 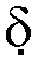 1.16 (t, J=7.2 Hz, 3H), 3.07-3.22 (m, 2H), 6.60 (d, J=9.3 Hz, 2H), 7.15-7.25 (m, 1H), 7.97 (d, J=9.3 Hz, 2H). MS: 167.0 [C8H10N2O2+H]⁺.

### Example 5: Synthesis of Dimethyl-(4-nitro-phenyl)-amine

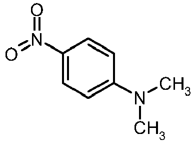

Dimethyl-(4-nitro-phenyl)-amine was purchased from Alfa Aesar. CAS [100-23-2]. Catalogue # L0040G. Batch # 10107319. 1H NMR (300 MHz, CHCl3-d): δ 3.07 (s, 6H), 6.75 (d, J=9.5 Hz, 2H), 8.03 (d, J=9.5 Hz, 2H). MS: 167.0 [C8H10N2O2+H]⁺.

### Example 6: Synthesis of (4-Nitro-phenyl)-propyl-amine

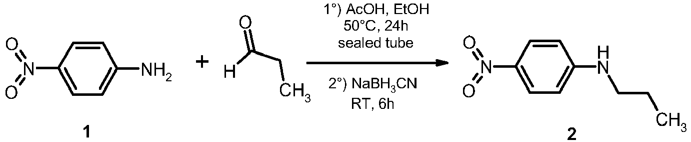

To a solution of 4-nitroaniline **1** (3.00 g, 1.0 eq) in 70 mL of absolute ethanol were added propionaldehyde (1.90 g, 1.2 eq) and glacial acetic acid (2.48 mL, 2.0 eq). The reaction mixture was heated at 70°C for 24 hours in a sealed tube. After cooling down to room temperature, sodium cyanoborohydride (2.18 g, 1.6 eq) was added in portions and the mixture was stirred at room temperature for 18 hours. After water addition (50 mL), the mixture was extracted with ethyl acetate (3x100 mL), dried over sodium sulphate and solvents were evaporated under vaccum. The residue was adsorbed on silica gel and purified by flash chromatography (cyclohexane/ethyl acetate 100/0 to 100/0 followed by cyclohexane/ethyl acetate 100/0 to 80/20) affording 0.87 g of compound **2** as a yellow powder; yield 0.78 g (4.33 mmol, 22 %).
1 H NMR (300 MHz, DMSO-d6): δ 0.89 (t, J=7.3 Hz, 3H), 1.56 (sext, J=7.3 Hz, 2H), 3.02-3.15 (m, 2H), 6.61 (d, J=9.3 Hz, 2H), 7.20-7.32 (m, 1 H), 7.96 (d, J=9.3 Hz, 2H). MS: 181.0 [C9H12N202+H]⁺.

### Example 7: Synthesis of Isopropyl-(4-nitro-phenyl)-amine

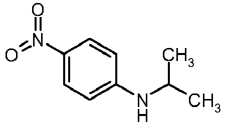

Isopropyl-(4-nitro-phenyl)-amine was purchased from Alfa Aesar. CAS [25186-43-0]. Catalogue # H52375. Batch # A05X026. 1H NMR (300 MHz, CHCl3-d): 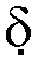 1.15 (d, J=6.4 Hz, 6H), 3.58-3.77 (m, 1 H), 6.60 (d, J=9.3 Hz, 2H), 7.13 (d, J=7.5 Hz 1 H), 7.96 (d, J=9.3 Hz, 2H). MS: 191.0 [C9H12N2O2+H]⁺.

### Example 8: Synthesis of 2-(4-Nitro-phenylamino)-ethanol

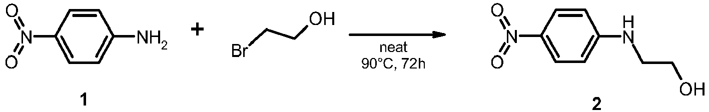

4-Nitroaniline **1** (2.00 g, 1.0 eq) was mixed in 2-bromoethanol (0.71 mL, 0.7 eq) in a sealed tube. The reaction mixture was stirred at 90°C for 72 hours. After dilution with ethyl acetate (50 mL) and (1 N) sodium hydroxide solution (50 mL), the mixture was extracted with ethyl acetate (3x100 mL), washed with (1N) sodium hydroxide solution (2x50 mL), washed with water (50 mL), dried over sodium sulphate and solvents were evaporated under vaccum. The residue was adsorbed on silica gel and purified by flash chromatography (cyclohexane/ethyl acetate 100/0 to 100/0 followed by cyclohexane/ethyl acetate 100/0 to 30/70) affording compound **2** as a yellow powder; yield 1.13 g (6.2 mmol, 61 %).
1H NMR (300 MHz, DMSO-d6): δ 3.15-3.27 (m, 2H), 3.49-3.60 (m, 2H), 4.78 (t, J=5.3 Hz, 1 H), 6.64 (d, J=9.3 Hz, 2H), 7.20-7.35 (m, 1 H), 7.96 (d, J=9.3 Hz, 2H). MS: 183.0 [C8H10N203+H]⁺.

### Example 9: Synthesis of 3-(4-Nitro-phenylamino)-propan-1-ol

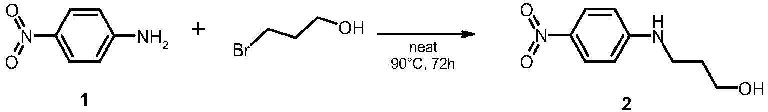

4-Nitroaniline **1** (5.00 g, 1.0 eq) was mixed in 3-bromopropanol (2.61 mL, 0.7 eq) in a sealed tube. The reaction mixture was stirred at 90°C for 18 hours. After dilution with ethyl acetate (50 mL) and (1 N) sodium hydroxide solution (50 mL), the mixture was extracted with ethyl acetate (3x100 mL), washed with water (50 mL), dried over sodium sulphate and solvents were evaporated under vaccum. The residue was adsorbed on silica gel and purified by flash chromatography (cyclohexane/ethyl acetate 100/0 to 100/0 followed by cyclohexane/ethyl acetate 100/0 to 30/70) affording of compound **2** as a yellow powder; yield 3.29 g (16.76 mmol, 58 %).
1H NMR (300 MHz, DMSO-d6): δ 1.69 (quint, J=6.3 Hz, 2H), 3.12-3.24 (m, 2H), 3.41-3.53 (m, 2H), 4.51 (t, J=5.1 Hz, 1 H), 6.61 (d, J=9.3 Hz, 2H), 7.24 (t, J=5.2 Hz, 1 H), 7.96 (d, J=9.3 Hz, 2H). MS: 197.0 [C9H12N203+H]⁺.

### Example 10: Synthesis 4-Nitro-N-(Pyridin-2-ylmethyl)aniline

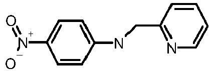

4-Nitro-N-(Pyridin-2-ylmethyl)aniline was purchased from AURORA FINE CHEMICALS. CAS [408365-67-3]. Catalogue # A00.198.769. Batch # 1108894.

### Example 11: Synthesis of 3-((4-Nitro-phenylamino)-methyl)-benzoic acid

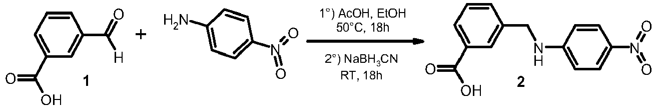

To a solution of 3-carboxybenzaldehyde **1** (2.17 g, 1.0 eq) in 100 mL of ethanol (dry) were added 4-nitroaniline (2.00 g, 1.0 eq) and glacial acetic acid (1.66 mL, 2.0 eq). The reaction mixture was heated at 50°C for 18 hours. After cooling to room temperature, sodium cyanoborohydride (1.46 g, 1.6 eq) was added in portions and the mixture was stirred at room temperature for 18 hours. After solvent evaporation under vacuum, the residue was triturated in water (100 mL), filtered off and washed with dichloromethane/methanol (95/5) mixture (10 mL) to afford compound 2 as a yellow solid; Yield 2.18 g (8.0 mmol, 55 %).
1H NMR (300 MHz, DMSO-d6): δ 4.48 (d, J=6.1 Hz, 2H), 6.66 (d, J=9.3 Hz, 2H), 7.46 (t, J=7.7 Hz, 1 H), 7.58 (d, J=7.7 Hz, 1 H), 7.77-8.00 (m, 5H), 12.95 (s, 1 H).
MS: 273.1 [C14H12N204+H]⁺.

## Claims

1. Use of at least one compound as defined by formula (I) or a salt thereof as an active compound in animal feeding for reducing the formation of methane emanating from the digestive activities of ruminants. 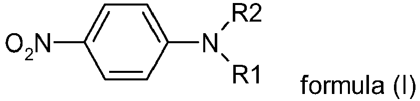 wherein R¹ and R² independently of each other represent H, -CH₃, or-CH₂R³, and wherein R³ is a saturated or unsaturated, linear, branched or cyclic C₁-C₈ -hydrocarbon group, optionally substituted with 1 to 3 groups selected from -OH, -NH₂, -COOH, and wherein one or two of the carbon atoms in the C₁-C₈ hydrocarbon is optionally substituted by a nitrogen or an oxygen atom.

2. Use according to claim 1, wherein R¹ is H or-CH₃.

3. Use according to claim 1 or 2, wherein R¹ is H or -CH₃, R² is H, -CH₃, or-CH₂R³, and R³ is a saturated, linear, or branched C₁-C₃ -hydrocarbon group.

4. Use according to any of claims 1 to 3, wherein the compound of formula (I) is selected from the group of compounds comprising: (4-nitro-phenyl)-amine, methyl-(4-nitro-phenyl)-amine, ethyl-(4-nitro-phenyl)-amine, dimethyl-(4-nitrophenyl)-amine, (4-Nitro-phenyl)-propyl-amine, isopropyl-(4-nitro-phenyl)-amine, 2-(4-Nitro-phenylamino)-ethanol, 3-(4-Nitro-phenylamino)-propan-1-ol, 4-Nitro-N-(Pyridin-2-ylmethyl)aniline, 4-Nitro-N-(Pyridin-2-ylmethyl)aniline, and 3-((4-Nitro-phenylamino)-methyl)-benzoic acid.

5. Use according to any of claims 1 to 4, wherein the at least one compound of formula (I), or a salt thereof is combined with at least one additional active substance selected from the group consisting of diallyl disulfide, garlic oil, allyl isothiocyanate, deoxycholic acid, chenodeoxycholic acid and derivatives thereof.

6. Use according to any of claims 1 to 5, wherein the ruminant animal is selected from the group consisting of: cattle, goats, sheep, American Bison, European bison, yaks, and water buffalo.

7. Use according to any of claims 1 to 6, wherein the methane production in ruminants calculated in liters per kilogram of dry matter intake is reduced by at least 10 % when measured in metabolic chambers.

8. Use according to any of claims 1 to 7, wherein the amount of the at least one active compound as defined in formula (I) administered to the ruminant animal is from 1 mg to 10 g per kg feed.

9. A feed composition or feed additive comprising at least one compound of formula (I) according to any of claims 1 to 4, the feed composition being a mineral premix, a vitamin premix, or a premix including vitamins and minerals or a bolus.

10. A method for reducing the production of methane emanating from the digestive activities of ruminants comprising orally administering a sufficient amount of at least one compound of formula (I), or a salt thereof to the animal, 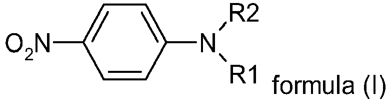 wherein R¹ and R² independently of each other represent H, -CH₃, or-CH₂R³, and wherein R³ is a saturated or unsaturated, linear, branched or cyclic C₁-C₈ -hydrocarbon group, optionally substituted with 1 to 3 groups selected from -OH, -NH₂, -COOH, and wherein one or two of the carbon atoms in the C₁-C₈ hydrocarbon is optionally substituted by a nitrogen or an oxygen atom.

11. A method according to claim 10, wherein the at least one compound of formula (I) is administered to the animal in combination with at least one additional active substance selected from the group consisting of diallyl disulfide, garlic oil, allyl isothiocyanate, deoxycholic acid, chenodeoxycholic acid and derivatives thereof.

12. A method according to claims 10 or 11, wherein the ruminant animal is selected from the group consisting of: cattle, goats, sheep, giraffes, American Bison, European bison, yaks, water buffalo, deer, camels, alpacas, llamas, wildebeest, antelope, pronghorn, and nilgai.

13. A method according to any of claims 10 to 12, wherein the amount of the at least one compound of formula (I) administered to the ruminant animal is from 1 mg to 10 g per kg feed.

14. A method according to any of claims 10 to 13, wherein the methane production in ruminants calculated in liters per kilogram of dry matter intake is reduced by at least 10 % when measured in metabolic chambers.

## Patentansprüche

1. Verwendung von mindestens einer Verbindung wie durch die Formel (I) definiert oder eines Salzes davon als Wirkstoff in der Tierfütterung zum Reduzieren der Bildung von Methan, das aufgrund der Verdauungsaktivitäten von Wiederkäuern entsteht, 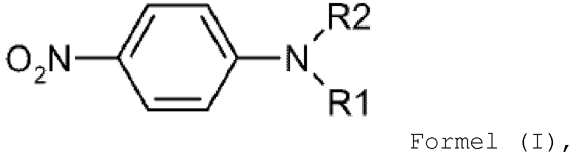 wobei R¹ und R² unabhängig voneinander H, -CH₃ oder -CH₂R₃ bedeuten und wobei R³ eine gesättigte oder ungesättigte geradkettige, verzweigte oder ringförmige C₁-C₈-Kohlenwasserstoffgruppe ist, die gegebenenfalls durch 1 bis 3 Gruppen ausgewählt aus -OH, -NH₂, -COOH substituiert ist und wobei eines oder zwei der Kohlenstoffatome in dem C₁-C₈-Kohlenwasserstoff gegebenenfalls durch ein Stickstoff- oder ein Sauerstoffatom substituiert ist/sind.

2. Verwendung nach Anspruch 1, wobei R¹ H oder -CH₃ bedeutet.

3. Verwendung nach Anspruch 1 oder 2, wobei R¹ H oder -CH₃ bedeutet, R² H, -CH₃ oder -CH₂R₃ bedeutet und R³ eine gesättigte geradkettige oder verzweigte C₁-C₃-Kohlenwasserstoffgruppe ist.

4. Verwendung nach einem der Ansprüche 1 bis 3, wobei die Verbindung der Formel (I) aus der Gruppe von Verbindungen ausgewählt ist, die Folgendes umfasst: (4-Nitrophenyl)amin, Methyl-(4-nitrophenyl)amin, Ethyl-(4-nitrophenyl)amin, Dimethyl-(4-nitrophenylamin), (4-Nitrophenyl)propylamin, Isopropyl-(4-nitrophenyl)amin, 2-(4-Nitrophenyl-amino)ethanol, 3-(4-Nitrophenylamino)propan-1-ol, 4-Nitro-N-(pyridin-2-ylmethyl)anilin, 4-Nitro-N-(pyridin-2-ylmethyl)anilin und 3-((4-Nitrophenylamino)methyl)benzoesäure.

5. Verwendung nach einem der Ansprüche 1 bis 4, wobei die mindestens eine Verbindung der Formel (I) oder ein Salz davon mit mindestens einem zusätzlichen Wirkstoff, ausgewählt aus der Gruppe bestehend aus Diallyldisulfid, Knoblauchöl, Allylisothiocyanat, Desoxycholsäure, Chenodesoxycholsäure und Derivaten davon kombiniert wird.

6. Verwendung nach einem der Ansprüche 1 bis 5, wobei der Wiederkäuer aus der Gruppe bestehend aus Rindern, Ziegen, Schafen, Amerikanischem Bison, Wisent, Yaks und Wasserbüffel ausgewählt ist.

7. Verwendung nach einem der Ansprüche 1 bis 6, wobei die Methanproduktion in Wiederkäuern, berechnet in Litern pro Kilogramm Trockenmasseaufnahme, bei Messung in Stoffwechselkammern um mindestens 10% reduziert ist.

8. Verwendung nach einem der Ansprüche 1 bis 7, wobei die Menge des mindestens einen Wirkstoffs wie in Formel (I) definiert, die dem Wiederkäuer verabreicht wird, 1 mg bis 10 g pro kg Futter beträgt.

9. Futterzusammensetzung oder Futterzusatzstoff, umfassend mindestens eine Verbindung der Formel (I) nach einem der Ansprüche 1 bis 4, wobei die Futterzusammensetzung eine Mineralienvermischung, eine Vitaminvermischung oder eine Vermischung, die Vitamine und Mineralien beinhaltet, oder ein Bolus ist.

10. Verfahren zum Reduzieren der Bildung von Methan, das aufgrund der Verdauungsaktivitäten von Wiederkäuern entsteht, bei dem man eine ausreichende Menge an mindestens einer Verbindung der Formel (I) oder ein Salz davon dem Tier oral verabreicht, 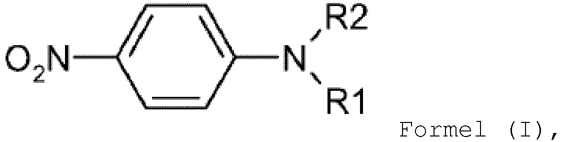 wobei R¹ und R² unabhängig voneinander H, -CH₃ oder -CH₂R₃ bedeuten und wobei R³ eine gesättigte oder ungesättigte geradkettige, verzweigte oder ringförmige C₁-C₈-Kohlenwasserstoffgruppe ist, die gegebenenfalls durch 1 bis 3 Gruppen ausgewählt aus -OH, -NH₂, -COOH substituiert ist und wobei eines oder zwei der Kohlenstoffatome in dem C₁-C₈-Kohlenwasserstoff gegebenenfalls durch ein Stickstoff- oder ein Sauerstoffatom substituiert ist/sind.

11. Verfahren nach Anspruch 10, wobei die mindestens eine Verbindung der Formel (I) dem Tier in Kombination mit mindestens einem zusätzlichen Wirkstoff, ausgewählt aus der Gruppe bestehend aus Diallyldisulfid, Knoblauchöl, Allylisothiocyanat, Desoxycholsäure, Chenodesoxycholsäure und Derivaten davon verabreicht wird.

12. Verfahren nach den Ansprüchen 10 oder 11, wobei der Wiederkäuer aus der Gruppe bestehend aus Rindern, Ziegen, Schafen, Giraffen, dem Amerikanischen Bison, dem Wisent, Yaks, Wasserbüffeln, Hirschen, Kamelen, Alpakas, Lamas, Gnus, Antilope, Pronghorn und Nilgai ausgewählt ist.

13. Verfahren nach einem der Ansprüche 10 bis 12, wobei die Menge der mindestens einen Verbindung der Formel (I), die dem Wiederkäuer verabreicht wird, 1 mg bis 10 g pro kg Futter beträgt.

14. Verfahren nach einem der Ansprüche 10 bis 13, wobei die Methanproduktion in Wiederkäuern, berechnet in Litern pro Kilogramm Trockenmasse-aufnahme, bei Messung in Stoffwechselkammern um mindestens 10% reduziert ist.

## Revendications

1. Utilisation d'au moins un composé tel que défini par la formule (I) ou un sel de celui-ci en tant que composé actif en alimentation animale pour réduire la formation de méthane émanant des activités digestives de ruminants. 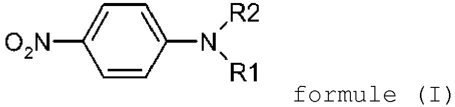 dans lequel R¹ et R² indépendamment l'un de l'autre représentent H, -CH₃, ou- CH₂R³, et dans lequel R³ est un groupe hydrocarbure en C₁-C₈ saturé ou insaturé, linéaire, ramifié ou cyclique, facultativement substitué par 1 à 3 groupes choisis parmi -OH, -NH₂,-COOH, et dans lequel un ou deux des atomes de carbone dans l'hydrocarbure en C₁-C₈ est facultativement substitué par un atome d'azote ou d'oxygène.

2. Utilisation selon la revendication 1, dans laquelle R¹ est H ou -CH₃.

3. Utilisation selon la revendication 1 ou 2, dans laquelle R¹ est H ou -CH₃, R² est H, -CH₃, ou -CH₂R³, et R³ est un groupe hydrocarbure en C₁-C₃ saturé, linéaire, ou ramifié.

4. Utilisation selon l'une quelconque des revendications 1 à 3, dans laquelle le composé de formule (I) est choisi dans le groupe de composés comprenant : (4-nitro-phényl)-amine, méthyl-(4-nitro-phényl)-amine, éthyl-(4-nitro-phényl)-amine, diméthyl-(4-nitro-phényl)-amine, (4-nitro-phényl)-propyl-amine, isopropyl-(4-nitro-phényl)-amine, 2-(4-nitro-phénylamino)-éthanol, 3-(4-nitro-phénylamino)-propan-1-ol, 4-nitro-N-(pyridin-2-ylméthyl)aniline, 4-nitro-N-(pyridin-2-ylméthyl)aniline, et acide 3-((4-nitro-phénylamino)-méthyl)-benzoïque.

5. Utilisation selon l'une quelconque des revendications 1 à 4, dans laquelle l'au moins un composé de formule (I), ou un sel de celui-ci est combiné avec au moins une substance active additionnelle choisie dans le groupe constitué des disulfure de diallyle, huile d'ail, isothiocyanate d'allyle, acide désoxycholique, acide chénodésoxycholique et des dérivés de ceux-ci.

6. Utilisation selon l'une quelconque des revendications 1 à 5, dans laquelle l'animal ruminant est choisi dans le groupe constitué des : bovins, chèvres, moutons, bison d'Amérique du Nord, bison d'Europe, yaks, et buffle d'eau.

7. Utilisation selon l'une quelconque des revendications 1 à 6, dans laquelle la production de méthane chez des ruminants calculée en litres par kilogramme d'ingestion de matière sèche est réduite d'au moins 10 % lorsqu'elle est mesurée dans des chambres métaboliques.

8. Utilisation selon l'une quelconque des revendications 1 à 7, dans laquelle la quantité de l'au moins un composé actif comme défini dans la formule (I) administrée à l'animal ruminant est de 1 mg à 10 g par kg d'aliment pour animaux.

9. Composition d'aliment pour animaux ou additif d'aliment pour animaux comprenant au moins un composé de formule (I) selon l'une quelconque des revendications 1 à 4, la composition d'aliment pour animaux étant un prémélange de minéraux, un prémélange de vitamines, ou prémélange comprenant des vitamines et des minéraux ou un bolus.

10. Procédé de réduction de la production de méthane émanant des activités digestives de ruminants comprenant l'administration orale d'une quantité suffisante d'au moins un composé de formule (I), ou un sel de celui-ci à l'animal, 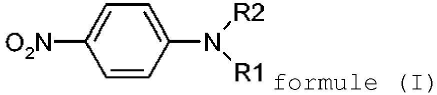 dans lequel R¹ et R², indépendamment l'un de l'autre, représentent H, -CH₃, ou - CH₂R³, et dans lequel R³ est un groupe hydrocarbure en C₁-C₈ saturé ou insaturé, linéaire, ramifié ou cyclique, facultativement substitué par 1 à 3 groupes choisis parmi -OH, -NH₂,-COOH, et dans lequel un ou deux des atomes de carbone dans l'hydrocarbure en C₁-C₈ est facultativement substitué par un atome d'azote ou d'oxygène.

11. Procédé selon la revendication 10, dans lequel l'au moins un composé de formule (I) est administré à l'animal en combinaison avec au moins une substance active additionnelle choisie dans le groupe constitué des disulfure de diallyle, huile d'ail, isothiocyanate d'allyle, acide désoxycholique, acide chénodésoxycholique et des dérivés de ceux-ci.

12. Procédé selon les revendications 10 ou 11, dans lequel l'animal ruminant est choisi dans le groupe constitué des : bovins, chèvres, moutons, girafes, bison d'Amérique du Nord, bison d'Europe, yaks, buffle d'eau, cerf, chameaux, alpagas, lamas, gnous, antilopes, pronghorn et nilgai.

13. Procédé selon l'une quelconque des revendications 10 à 12, dans lequel la quantité de l'au moins un composé de formule (I) administrée à l'animal ruminant est de 1 mg à 10 g par kg d'aliment pour animaux.

14. Procédé selon l'une quelconque des revendications 10 à 13, dans lequel la production de méthane chez des ruminants calculée en litres par kilogramme d'ingestion de matière sèche est réduite d'au moins 10 % lorsqu'elle est mesurée dans des chambres métaboliques.
